# EUROPEAN PATENT SPECIFICATION

(11) **EP 2 893 934 B1**
(45) Date of publication and mention of the grant of the patent: **03.10.2018**
(21) Application number: 15150709.2
(22) Date of filing: 09.01.2015
(51) Int. Cl.: A61K 36/67, A61P 33/02, A61K 38/01, A61K 35/60, A23K 20/10, A23K 20/20, A23K 50/40

(54) **PRODUCT FOR FEEDING DOGS IN ORDER TO PROTECT THEM FROM LEISHMANIASIS**
HUNDERFUTTER UM HUNDE VOR LEISHMANIOSE ZU SCHÜTZEN
NOURRITURE POUR CHIENS POUR LES PROTEGER DE LA LEISMANIOSE

(30) Priority: 09.01.2014 IT PD20140002
(43) Date of publication of application: 15.07.2015
(73) Proprietor: Sanypet S.p.a., 35023 Bagnoli di Sopra (PD) (IT)
(72) Inventor: Canello, Sergio, I-35037 Villa di Teolo (PD) (IT); Guidetti, Gianandrea, I-42121 Reggio Emilia (RE) (IT)
(74) Representative: Gallo, Luca

(56) References cited:
- EP-A1- 2 526 781
- WO-A1-2009/019593
- FERREIRA C ET AL: "Leishmanicidal effects of piperine, its derivatives, and analogues on", PHYTOCHEMISTRY, PERGAMON PRESS, GB, vol. 72, no. 17, 2 August 2011 (2011-08-02), pages 2155-2164, XP028307658, ISSN: 0031-9422, DOI: 10.1016/J.PHYTOCHEM.2011.08.006 [retrieved on 2011-08-08]
- KAPIL A: "Piperine: a potent inhibitor of Leishmania donovani promastigotes in vitro", PLANTA MEDICA, THIEME VERLAG, DE, vol. 59, no. 5, 1 October 1993 (1993-10-01), page 474, XP002423245, ISSN: 0032-0943, DOI: 10.1055/S-2006-959737
- HEAD E ET AL: "A combination cocktail improves spatial attention in a canine model of human aging and Alzheimer's disease", JOURNAL OF ALZHEIMER'S DISEASE 2012 IOS PRESS NLD, vol. 32, no. 4, 2012, pages 1029-1042, XP009180148, ISSN: 1387-2877
- ANONYMOUS: "Super Premium Junior Dog Food", GNPD; MINTEL, June 2012 (2012-06), XP002717557,
- M. M. Kane ET AL: "The Role of IL-10 in Promoting Disease Progression in Leishmaniasis", THE JOURNAL OF IMMUNOLOGY, vol. 166, no. 2, 15 January 2001 (2001-01-15), pages 1141-1147, XP055286676, US ISSN: 0022-1767, DOI: 10.4049/jimmunol.166.2.1141

## Description

### Field of application

The present invention regards a product for feeding dogs suitable for performing an immunomodulation action as adjuvant in a standard pharmacological therapy for the treatment of canine leishmaniasis, according to the preamble of the independent claim No. 1.

The product, subject of the present invention, it intended to be employed for feeding dogs affected by leishmaniasis, in association with a suitable pharmacological therapy, for the purpose of limiting the degenerative effects of such pathology. Such product is also adapted to be administered to healthy dogs, in order to improve their immune response and reduce the risk that leishmaniasis infection in such dogs assume symptomatic character.

The invention is therefore inserted in the industrial field for producing products for feeding and treating animals of domestic type, and in particular dogs.

### State of art

As is known, leishmaniasis is a systemic pathology that afflicts dogs in particular; it is caused by the leishmania parasite. Such pathology, carried by insects, is endemic in Mediterranean countries and constitutes a serious veterinary problem, given that the diagnosis of such pathology is complicated to carry out and that the therapies currently available for treating such pathology are only partially effective and in any case are rarely able to be resolutive.

The course and gravity of leishmaniasis can vary greatly from one subject to another, such that the same disease can be asymptomatic in some subjects and can instead evolve into a clearly symptomatic disease in other subjects.

In recent years, many studies have been undertaken in the veterinary field aimed to identify the physical characteristics that mark infected subjects which nevertheless do not develop the classic symptoms of the leishmania pathology, with respect to the subjects which instead have the disease in symptomatic form.

In particular, such studies have brought to light that the immune response plays a very import role in the course of the disease and in the response of subjects to pharmacological therapies, in particular identifying the cell-mediated immune response (Th1), rather than the humoral or antibody immune response (Th2), as that which is more adapted for preventing a symptomatic course of the disease.

Given the fact that it is impossible to eliminate the parasite, currently available pharmacological therapies therefore aim to decrease the parasitic load in the animal and/or to facilitate the cell-mediated immune response of Th1 type.

More in detail, it has been verified that in dogs, as in humans, resistance to the disease seems to be associated with an immune response of mixed Th1-Th2 type, with predominance of cytokines Th1, while the development of the disease seems to be associated with a reduced production of cytokines, in particular of Th1 type. However, the factors that induce a mainly Th1 or Th2 response in the different subjects are not entirely known at the moment, given that only hypotheses have been formulated on the matter up to now. Some factors appear in particular to depend on the host; others, however, appear to depend on the parasite and its activity.

Generally, though, the specific immune response of the subject that has contracted the leishmania infection, whether mainly of Th1 type or of Th2 type, should be considered immutable over time.

As has in fact been demonstrated by Ferrer L. in the article "Simultaneous presentation of leishmaniasis and other infectious disease: clinical approach and mechanism", from the Proceedings of the International Congress on Canine Leishmaniasis, Naples, 2004 (pp. 37-38), the verification of some conditions can trigger changes in the immune response, such that in particular asymptomatic infected subjects can suddenly develop the disease symptoms.

In particular, among the decisive conditions causing the appearance of symptoms, immunodepression states are recognized, due for example to radiotherapies, to chemotherapies or to infections from Ehrlichia, from Babesia, from Hepatozoon canis, from Dirofilaria and from other pathogenic agents. Indeed, an immunodepressed organism has a harder time opposing the protozoa of leishmania, such that a quick reproduction thereof is facilitated, and all the primary and secondary negative effects induced by such protozoa in the different organs are enhanced.

An important aspect of the course of the disease in symptomatic clinical form is the appearance of alterations of blood protein content, of pathologies from immunocomplexes and from autoantibodies, as well as of immunodepression, determined by a continuous stress of the competent immune cells, with consequent immune disequilibrium, characterized by hyperstimulation of the humoral response and irregularities of the cell-mediated response.

In particular the pathologies from immunocomplexes are tied to the deposit of immunocomplexes in filter zones of the organism of the host (mainly on the basal membrane of the renal glomerule), becoming responsible for collateral pathologies such as hemorrhages, nervous disturbances, mucocutaneous ulcerations and in particular inflammations that affect different organs and/or tissues, such as vasculitis, interstitial pneumonitis, polyarthritis, uveitis, synovitis, glomerulonephritis, up to kidney failure, which constitutes the first cause of death of leishmaniasis dogs.

In the veterinary field, therefore, there is very much the need to develop therapeutic or adjuvant products that allow optimizing the immune response of the dogs that contract the leishmaniasis infection, as well as limiting the development of collateral pathologies to leishmaniasis or at least containing the effects of such pathologies.

In addition, in the field of production of products for feeding and treating animals, there has for some time been the need to provide specific products dedicated for feeding animals whose immune system is lacking or exhausted by pathologies, such as in particular leishmaniasis; such animals require a controlled diet adapted in particular to contain the trigger and propagation of inflammatory phenomena.

The patent WO 2009/019593 describes a product for feeding animals comprising food croquettes externally sprayed with fats or appetizing substances (such as protein hydrolysates extracted from chicken liver), and croquettes for therapeutic purposes comprising a mixture of powder ingredients compacted by means of dry forming. In particular, the ingredients of the croquettes for therapeutic purposes comprise thickener components (such as basic phosphate calcium, corn starch, etc.), a hydrophilic mineral and one or more therapeutic substances (such as vitamin C, lysozime, bioflavonoids, etc.) for treating animals.

The patent EP 2526781 describes a product for feeding animals comprising croquettes for feeding purposes and tablets for therapeutic purposes formed by ingredients such as fats, carbohydrates, fibers and ashes.

The food products for animals of known type described in the aforesaid patents WO 2009/019593 and EP 2526781 do not comprise nutritional and therapeutic ingredients capable of preventing or limiting the development of pathologies due to leishmaniasis.

The scientific articles "Leishmanicidal effects of piperine, its derivatives, and analogues on" by Ferreria et al., Phvtochemistry, vol. 72, No. 17 (2011), pp. 2155-2164, and "Piperine: a potent inhibitor of Leishmania donovani promastigotes in vitro" by Kapil, Planta Medica, vol. 59, No. 5 (1993), p. 474, treat clinical studies relative to the effects of piperine in therapeutic treatments of leishmaniasis.

The scientific article "A combination cocktail improves spatial attention in a canine model of human aging and Alzheimer's disease" by Head et al., Journal Of Alzheimer's Disease, vol. 31, No. 4 (2012), pp. 1029-1042, describes some tests executed in the laboratory on groups of dogs in order to verify the effects of piperine on the pathologies tied with Alzheimer.

Nevertheless, such articles do not in any way confront the problem of developing food products for dogs adapted for preventing or treating leishmaniasis.

### Presentation of the invention

In this situation, the problem underlying the present invention is to provide a product for feeding dogs in order to protect them from leishmaniasis, which is capable of improving the immune response of the dogs to which it is administered. A further object of the present invention is to provide a product for feeding dogs in order to protect them from leishmaniasis, which is able to contain the inflammatory reactions caused by the activation of the immune system in the subjects affected by leishmaniasis.

Another object of the present invention is to provide a product for feeding dogs in order to protect them from leishmaniasis, which is able to improve the balance between the immune responses of Th1 and Th2 type in the subjects affected by leishmaniasis.

A further object of the present invention is to provide a product for feeding dogs in order to protect them from leishmaniasis, which is able to reinforce the immune defenses of healthy subjects.

Another object of the present invention is to provide a product for feeding dogs in order to protect them from leishmaniasis, which is well tolerated by the dogs themselves and minimally allergenic.

A further object of the present invention is to provide a product for feeding dogs in order to protect them from leishmaniasis, which ensures an optimal assimilability of the nutritional substances contained therein.

Another object of the present invention is to provide a product for feeding dogs in order to protect them from leishmaniasis, which is tasty.

### Brief description of the drawings

The technical characteristics of the invention, according to the aforesaid objects, can be clearly seen from the contents of the below-reported claims and the advantages thereof will be clearer in the following detailed description, made with reference to the enclosed table and figures, which refer to a merely exemplifying and non-limiting embodiment, wherein:

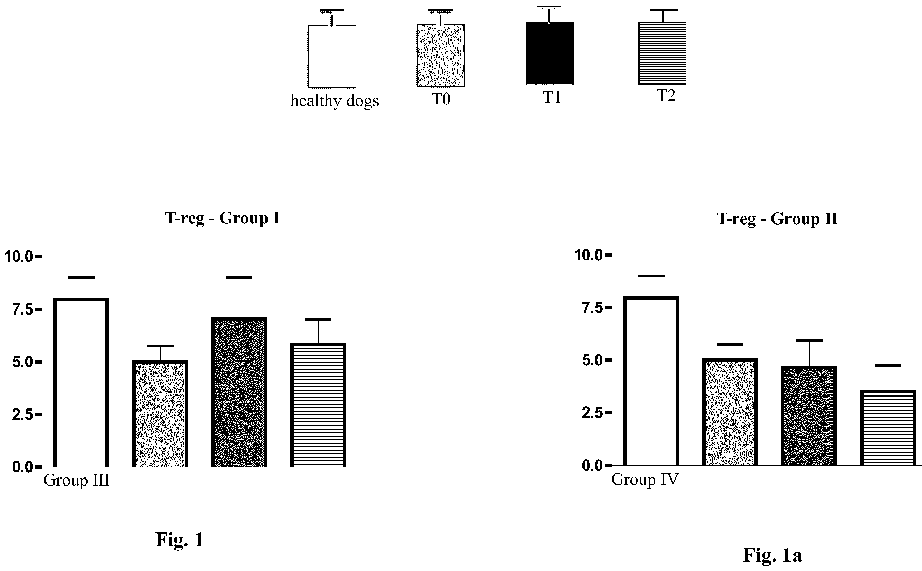

- Figures 1 and 1a show two first graphs bearing the average percentage of regulatory cells T present in the organism of dogs respectively fed with the product according to the present invention and with a standard diet in the scope of a first clinical trial, at the time of the start of the trial (T0), after three months of treatment (T1) and after six months of treatment (T2);

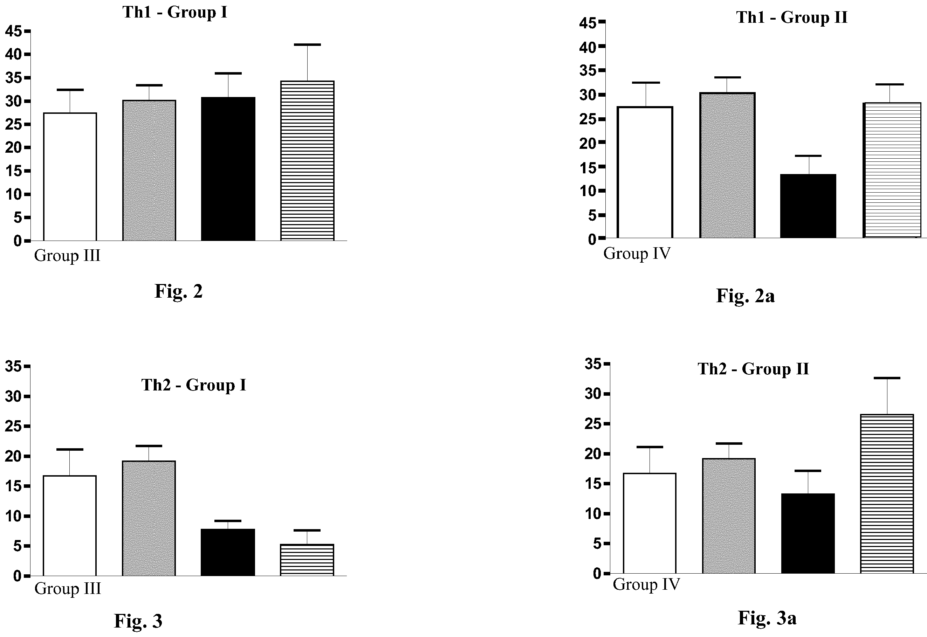

- Figures 2 and 2a show two second graphs bearing the percentage of cells Th1 present in the organism of dogs respectively fed with the product according to the present invention and with a standard diet in the scope of the aforesaid first clinical trial, at the time of the start of the trial (T0), after three months of treatment (T1) and after six months of treatment (T2);

- Figures 3 and 3a show two third graphs bearing the percentage of cells Th2 present in the organism of dogs respectively fed with the product according to the present invention and with a standard diet in the scope of the aforesaid first clinical trial, at the time of the start of the trial (T0), after three months of treatment (T1) and after six months of treatment (T2);

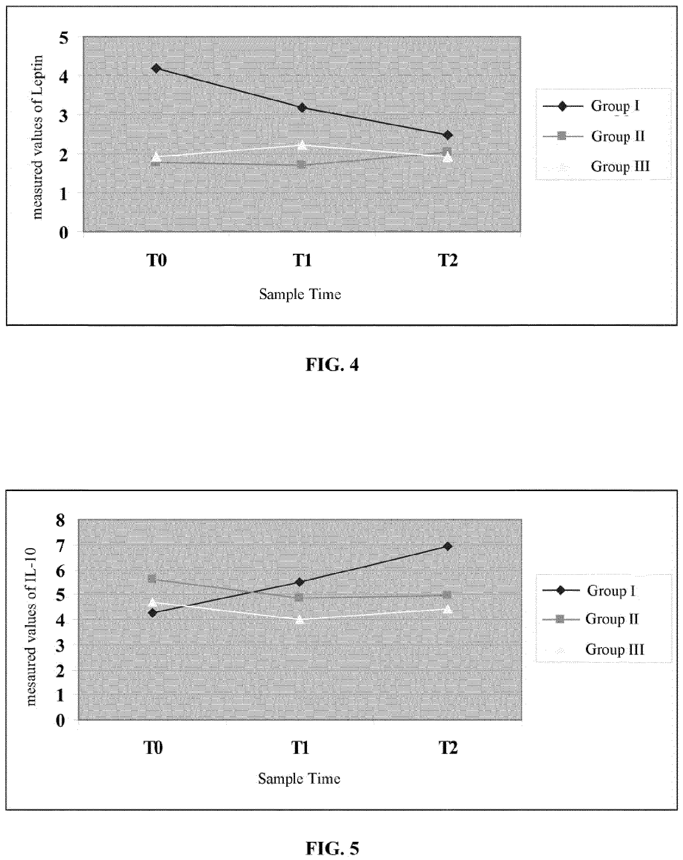

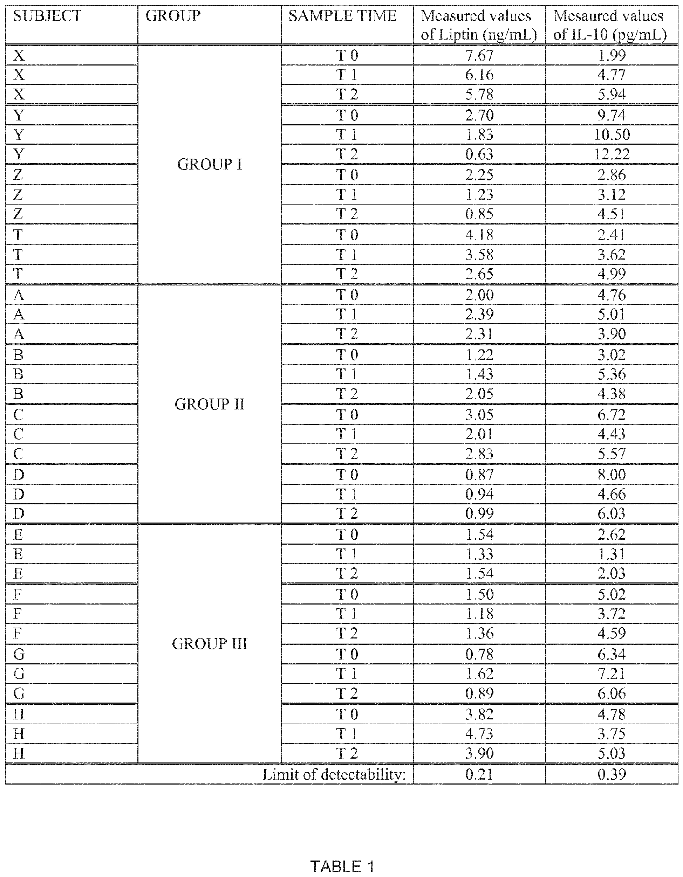

- Figure 4 shows a fourth graph reporting the concentration of serum leptin measured in dogs affected by leishmaniasis divided into three different groups receiving different foods, including Group I fed with the product for feeding animals according to the present invention and the Groups II and III fed with the product for feeding animals according to the invention respectively lacking the protein hydrolysates and the phytotherapeutic extract of Piper nigrum, in the scope of a second clinical trial;
- Figure 5 shows a fifth graph reporting the concentration of interleukin 10 measured in the serum of the same dogs, in the scope of the aforesaid second clinical trial;
- Table 1 reports as an example the values of leptin and interleukin 10 that were measured in the serum of several of the dogs subjected to the aforesaid second clinical trial.

### Detailed description

The product, subject of the present invention, is intended to be employed for feeding dogs in order to protect the same from leishmaniasis.

With the expression "in order to protect them from leishmaniasis" it is intended herein to indicate that the product according to the invention is adapted both to preventively protect the dogs that assume it, i.e. before the same possibly contract leishmaniasis, improving the immune defenses of such dogs and thus decreasing the probabilities that the same contract and develop the disease in symptomatic form, and to protect the organism of dogs already affected by leishmaniasis, improving the immune response of such dogs and preventing the same from developing the disease in symptomatic form or making the disease in symptomatic form revert towards an asymptomatic form.

More in detail, the present product is adapted to be administrated to infected subjects, i.e. which have already contracted the leishmaniasis infection, together with a pharmacological therapy in order to facilitate an optimal immune response in such subjects that allows limiting the symptoms of leishmaniasis and the onset of collateral pathologies. The same product is also adapted to be administrated to healthy dogs, i.e. which have not contracted the leishmaniasis infection, in order to strengthen the immune defenses of such dogs and to facilitate in the latter a quick and optimal immune response if the same contract the infection.

The product for feeding dogs in accordance with the invention comprises a nutritional matrix and one or more phytotherapeutic extracts.

In accordance with the idea underlying the present invention, the nutritional matrix comprises one or more protein hydrolysates, which are contained in the product for feeding dogs in a percentage by weight comprised between 7% and 14%, the phytotherapeutic extracts provided in the formulation of the product comprise a phytotherapeutic extract of Piper nigrum, present in the product for feeding dogs according to the invention in a percentage by weight comprised between 0.0015% and 0.0035%.

As is better explained hereinbelow, clinical studies carried out on dogs naturally affected by leishmaniasis in symptomatic clinical form have demonstrated that the administration of a diet based on the product for feeding dogs in accordance with the invention, in association with a classic anti-leishmaniasis pharmacological therapy, in particular constituted by meglumine antimoniate and allopurinol at dosages normally employed in clinical practice, has led to a significant improvement in the general clinical conditions of the dogs that were in the study. Comparable clinical results were however not attained by the dogs which, in association with the same pharmacological therapy, were subjected to a diet regime based on a product for feeding dogs entirely similar to the product subject of the present invention, but lacking the protein hydrolysates or the phytotherapeutic extract of Piper nigrum.

The values of some biochemical parameters measured in the dogs subjected to the clinical trial at different times of the trial were in line with the abovementioned clinical evaluations.

More in detail, the subjects fed with the complete product in accordance with the present invention experienced a progressive decrease of the serum leptin quantity in accordance with a similarly progressive increase of the interleukin 10 (IL-10) quantity present in the serum.

Leptin is a hormone that performs a role of primary importance on the activity of the T lymphocytes and on the immune system in general. In particular, recent studies have demonstrated that there is an inverse ratio between the levels of plasmatic leptin and the percentages of circulating Treg lymphocytes, such that the decrease of the serum leptin quantity is associated with an increase of the percentage of circulating Treg lymphocytes, and such lymphocytes have a fundamental function in the maintenance of the homeostasis of the immune system and protect the tissues from the harmful action of inflammatory processes, being able to intervene directly in the inflammation site in order to suppress the pro-inflammatory function of the activated cells.

The Treg lymphocytes, in fact, are known for the capacity to depress the autoreactive immune responses, inhibiting the appearance of autoimmune phenomena.

On the other hand, the increase of the plasmatic leptin levels is correlated with a reduction of the percentage of Treg lymphocytes. A deficit of the Treg lymphocytes as well as a reduced functionality thereof cause autoimmune phenomena. In particular, a reduction of the percentage of Treg lymphocytes indicates a considerable decrease of cell-mediated regulation mechanisms; an inflammatory response is associated therewith, represented by increased peripheral levels of cytotoxic T cells.

On the basis of numerous studies conducted specifically on dogs affected by leishmaniasis pathology, the maintenance of an active immune cytotoxic response, with a reduction of the modulation mechanisms dependent on the Treg, was considered to be at the base of numerous immune-mediated pathologies, frequently encountered in the course of canine leishmaniasis (as reported for example in the articles: "Circulating immune complexes and renal function in canine leishmaniasis" by Lopez et al., 1996, J. Vet. Med. B 43, 469-474; "Levels of IgM and IgA circulating immune complexes in dogs with leishmaniasis" by Margarito et al., 1998, J. Vet. Med. B 45, 263-267; "Presence of anti-platelet IgM and IgG antibodies in dogs naturally infected by Leishmania infantum" by Terrazzano et al., 2006, Vet. Immunol. Immunopathol. 110, 331-337; "Secondary immune-mediated thrombocytopenia in dogs naturally infected by Leishmania infantum" by Cortese et al., 2009, Vet. Rec. 164, 778-782; and "Prevalence of anti-platelet antibodies in dogs naturally co-infected by Leishmania infantum and Ehrlichia canis" by Cortese et al., 2011, Vet. J. 188, 118-121).

Therefore, a decrease of the plasmatic leptin levels, as encountered in the dogs administrated the complete product according to the present invention, is directly correlated with a greater presence of circulating Treg lymphocytes and with a containment therefore of the inflammatory cell immune response, with a decrease of the risk of appearance of autoimmune diseases caused by the latter.

The increase of the quantities of circulating Treg lymphocytes is further demonstrated by the increase of the interleukin 10 concentration. Indeed, the latter is an anti-inflammatory cytokine mainly produced by monocytes and by regulatory T lymphocytes; hence interleukin 10 is a quantity indicator of such lymphocytes. Interleukin 10 is capable of inhibiting the synthesis of pro-inflammatory cytokines such as IFN-γ, IL-2, IL-3, TNF-α and GM-CSF, produced by cells such as macrophages and T helper lymphocytes of class 2.

The product in accordance with the present invention, due to the synergy between the protein hydrolysates and the phytotherapeutic extract of Piper nigrum contained therein, has demonstrated effective immunomodulation properties. In particular, based on the results briefly summarized above, which will be described more in detail hereinbelow, the subjects fed with the product according to the present invention have shown an increase of the cell-mediated immune response Th1 with a consequent improvement of the balance between the cell-mediated response Th1 and the humoral response Th2.

The synergistic action between the protein hydrolysates and the phytotherapeutic extract of Piper nigrum, present in the formulation of the product according to the present invention, is therefore able to modulate the immune response in the dogs subjected to a diet based on the product itself.

By means of the clinical trials conducted on dogs fed with the product according to the present invention and described hereinbelow, it was verified that the synergistic action between the protein hydrolysates and the phytotherapeutic extract of Piper nigrum is substantially expressed in a general improvement of the metabolism of the dogs, induced by the use of the highly-digestible protein hydrolysates as main protein source; such protein hydrolysates in the presence of Piper nigrum are further and surprisingly well-digested and assimilated by the dogs to which the product is administrated.

Protein hydrolysates are peptides with low molecular weight which are more easily digestible with respect to normal proteins. Given their low molecular weight, protein hydrolysates are incapable of stimulating allergic and/or inflammatory responses by the immune system of the dogs that assume the product according to the invention, not even if the immune system is particularly exhausted or altered as occurs for the dogs affected by pathologies, such as in particular leishmaniasis. The dogs which assume the product according to the present invention are therefore capable of assimilating proteins in an optimal manner by means of the protein hydrolysates, nevertheless without the latter stimulating inflammatory reactions by the immune system.

Due to the presence of phytotherapeutic extract of Piper nigrum, the assimilation of the proteins from the protein hydrolysates appears enhanced, with a facilitated increase of the lean mass and a decrease of the fat mass in the animal that assumes the product.

The phytotherapeutic properties of the Piper nigrum extract are due to piperine, which based on the results of the clinical trials has proven capable of promoting the absorption of nutritional principles, such as in particular the amino acids present in the protein hydrolysates. In addition, piperine is thermogenic and a stimulant of the metabolism, such that its assumption facilitates the consumption of the fat mass. The phytotherapeutic extract of Piper nigrum, with high concentration of piperine, thus improves the absorption of the protein hydrolysates, generating an increase of the metabolic action and sustaining the loss of fat mass.

Advantageously, the phytotherapeutic extract of Piper nigrum introduced in the product according to the invention is a dry extract titrated at 95% piperine.

The assumption of the product according to the present invention, due to the particular synergy that is established between the protein hydrolysates and the Piper nigrum extract, thus allows the dogs to reach a rebalancing of the ratio between their fat mass and their lean mass, in favor of the latter. In association with the rebalancing of such ratio, there is a decrease of the quantity of leptin produced and circulating in the organism, which as stated above is inversely proportional to the quantity of regulatory T cells and hence to the quantity of circulating IL-10. The increase of the number of TReg cells circulating in the organism allows an improved control of chronic diseases, such as leishmaniasis, and allows improving the balancing between Th1 and Th2 response in many infectious forms with exaggerated immune response, with restoration of good clinical conditions. In such infectious forms and diseases, indeed, a preponderant response of Th2 type would induce a significant clinical deterioration of the animal, which however would not be seen in the presence of a suitable quantity of Treg cells, such as that induced by the assumption of the product according to the invention. The product according to the present invention, administrated to dogs in particular affected by inflammatory diseases such as leishmaniasis, therefore carries out a role of immunomodulation of the immune response in such dogs, such to allow a more efficient and advantageous response of the immune system towards the pro-inflammatory pathogenic noxae.

The protein hydrolysates are comprised in the product for feeding dogs according to the invention in a percentage by weight comprised between 7% and 14%.

In accordance with the product, subject of the invention, the protein hydrolysates contained in the product comprise fish protein hydrolysate.

The fish protein hydrolysates, in fact, are particularly rich with Omega 3 and Omega 6 polyunsaturated fatty acids, both adapted to facilitate the reduction of inflammatory processes. Omega 3 fatty acids are also adapted to facilitate the reduction of allergic and autoimmune processes.

Preferably, the phytotherapeutic extract of Piper nigrum is comprised in the product for feeding dogs according to the invention in a percentage by weight comprised between 0.0020% and 0.0030% and advantageously equal to about 0.0025%.

Such percentage by weight of phytotherapeutic extract of Piper nigrum has in fact proven to be sufficiently high for ensuring the course of the synergistic action of the phytotherapeutic extract itself with the protein hydrolysates present in the product and at the same time sufficiently limited so as to prevent the phytotherapeutic extract from conferring to the overall product a taste or odor that is not very tasty/desirable for dogs, with the risk that the latter may consequently refuse to assume the product according to the present invention.

The product for feeding dogs according to the present invention, in addition to the phytotherapeutic extract of Piper nigrum, can advantageously also contain one or more further phytotherapeutic extracts selected in particular from among phytotherapeutic extracts of: melon, soy, echinacea, aloe, fermented papaya, pomegranate and green tea.

The phytotherapeutic extract of melon is in particular advantageously introduced into the formulation of the product for feeding dogs according to the present finding since it supplies superoxide dismutase (SOD). The latter is a strong antioxidant that contributes to the maintenance of the correct cellular oxidation-reduction state, being able to eliminate the radical of the oxygen by means of a dismutation reaction, transforming it into hydrogen peroxide. Oxygen radicals are continuously produced in small quantities in any organism in physiological conditions and are produced in significantly greater quantities in organisms under pathological conditions.

In addition - given that the superoxide ion O₂⁻, in the presence of transition metals, can be transformed into radical species of oxygen provided with high toxicity - the superoxide dismutase SOD, by eliminating the superoxide ions, carries out a detoxifying action.

The superoxide dismutase, introduced into the formulation of the product for feeding animals according to the invention by means of the phytotherapeutic extract of melon, has proven particularly suitable for facilitating the reduction of the inflammatory complications associated with leishmaniasis pathology, in addition to being suitable for enhancing the anti-microbe effects of the pharmacological therapy employed in the treatment of leishmaniasis.

The phytotherapeutic extract of soy is rich with isoflavones, which are phytoestrogens capable of performing a protective action of the organism and which naturally regulate the body hormone production.

The capacity of phytotherapeutic extracts of soy in reducing cholesterolemia has for some time been demonstrated. More in detail, numerous studies have shown that in particular the proteins of soy are capable of reducing the total cholesterol and the LDL cholesterol and of increasing the HDL cholesterol, i.e. the so-called "good" cholesterol. The soy proteins have also proven suitable for reducing the biosynthesis of triglycerides and free fatty acids, while the isoflavones have proven suitable for reducing the lipoprotein levels.

The introduction of phytotherapeutic extracts of soy and in particular of isoflavones of soy in the formulation of the product for feeding dogs according to the present invention is adapted to further facilitate the decrease of the fat mass, in favor of the lean mass.

The phytotherapeutic extract of echinacea, and in particular preferably of echinacea purpurea, has proven suitable for enhancing the immunomodulation properties of the food product, subject of the present invention. Indeed, such extract, in particular in combination with Piper nigrum and with the other phytotherapeutic extracts possibly present in the product according to the present invention, is capable of significantly increasing the number of total lymphocytes, stimulating the phagocytosis of the granulocytes and simultaneously reducing the T helper lymphocytes.

Echinacea is generally known for its non-specific immunostimulant action. Numerous experimental tests have in fact proven the capacity of echinacea in increasing the phagocytosis of the white blood cells, the differentiation of the immature white blood cells into mature white blood cells, the production and the activity of the macrophages, as well as the production of interferon and interleukins. In addition, echinacea is capable of carrying out an important anti-inflammatory action, increasing the production of the ACTH hormone adapted to stimulate the adrenal production of glucocorticoids, which perform a strong anti-inflammatory action.

Therefore, the introduction of the echinacea extract in the formulation of the product for feeding animals according to the invention is capable of enhancing the immunomodulation and anti-inflammatory properties of the product itself.

The phytotherapeutic extract of aloe, and in particular preferably of aloe vera, due in particular to its supply of fatty acids, of cholesterol and of campesterol, is capable of carrying out an immunostimulant and anti-inflammatory action.

Its introduction in the formulation of the product for feeding dogs, subject of the present invention, improves the immunomodulation properties of the latter and contributes to facilitating the development of a cell-mediated immune response. The plant and the fruit of the papaya are particularly rich with antioxidant substances and enzymes, such as in particular carotenoids, papain and chymopapain, which are capable of stimulating the immune system. The antioxidant content is particularly optimized in the extracted of fermented papaya, which is obtained via microbe fermentation of the entire fruit of the papaya.

The extract of fermented papaya has anti-inflammatory and antioxidant properties that are particularly useful for organisms weakened or affected by chronic diseases, such as those of dogs affected by leishmaniasis. The organisms of the subjects affected by chronic diseases in fact suffer an intense oxidative stress, due to an excess of free radicals that damage the cells of the organism. By exerting its antioxidant action, the extract of fermented papaya combats the free radicals, protecting the cells, and hence the organism, from damage correlated therewith. The phytotherapeutic extract of pomegranate is well-known to be provided with antioxidant properties. The antioxidant action of such extract, mainly mediated by the ellagitannins contained in the phytocomplex, is performed by means of the transformation of the free radicals of the oxygen into non-radical compounds, lacking reactivity and hence toxicity. The extract of pomegranate is also particularly rich with potassium, which has a diuretic effect and thus facilitates the draining of liquids and the elimination of toxins. The phytotherapeutic extract of pomegranate is also capable of carrying out an important anti-inflammatory action, in particular inhibiting the activation of the NF-kB factor and consequently simultaneously reducing the expression of the pro-inflammatory cytokine TNF-α. Therefore, the phytotherapeutic extract of pomegranate introduced in the product for feeding dogs according to the present invention is capable of enhancing the immunomodulation action performed by Piper nigrum in combination with the protein hydrolysates contained in the product, in particular facilitating the containment of the pro-inflammatory immune reaction and the protection of the tissues from the harmful action of the inflammatory processes initiated by the latter immune reaction.

The phytotherapeutic extract of green tea (Chamellia sinensis) is rich in epigallocatechins and in flavonoids and is capable of performing a strong radical scavenger action, having considerable antioxidant properties. Various clinical studies have shown that the phytotherapeutic extract of green tea is capable of performing a significant antioxidant action at the hepatic, plasmatic and nervous level, as well as overall in the organism, reducing the oxidative stress in the plasma and in the erythrocytes.

In addition, is has been verified that the phytotherapeutic extract of green tea has a strong suppressive effect on the synthesis of fats and the capacity to stimulate the thermogenesis of fats.

The phytotherapeutic extract of green tea, introduced in the formulation of the product for feeding dogs according to the invention, therefore contributes to facilitating the decrease of the fat mass and to facilitate a consequent use of the nutrients, increasing the lean mass.

In accordance with a particularly advantageous embodiment of the product for feeding dogs, such product comprises zinc, in addition to the phytotherapeutic extract of Piper nigrum and to the other phytotherapeutic extracts possibly present therein.

Zinc is a trace element essential for the correct functioning of the immune system. T lymphocytes are in particular the immune cells most sensitive to zinc depletion, and if the latter trace element is lacking, their multiplication and subsequent Th1 differentiation is compromised.

The subjects affected by chronic inflammatory conditions, such as those often verified in the case of parasitic diseases such as leishmaniasis in which a hepatic sequestration of zinc is verified, can show hypozincemia conditions.

The introduction of zinc, preferably in the form of highly bioavailable chelated zinc, in the formulation of the product for feeding dogs according to the present invention thus facilitates a favorable immune response. In particular, in the dogs affected by leishmaniasis, the integration of zinc - by stimulating the generation of the immune response Th1 - facilitates the resistance to the disease, slowing the progression thereof, given that the resistance to the disease is a function of the macrophages stimulated by the cytokines and IFN-γ produced by Th1 cells. On the other hand, it was verified that a deficiency of zinc causes a shift of the Th1/Th2 ratio, with the prevalent development of an immune response of humoral type, Th2.

Therefore the introduction of zinc in the formulation of the product for feeding dogs according to the present invention constitutes an important therapeutic aid in the control of leishmaniasis and is capable of performing a preventive function in the subjects more at risk of infection, decreasing the probabilities that the latter can develop the leishmaniasis pathology.

In accordance with a preferred embodiment of the product for feeding dogs according to the present invention, such product comprises food croquettes, preferably obtained via extrusion, and therapeutic tablets obtained via cold compression starting from powders. In particular, the croquettes obtained via extrusion are obtained starting from a mixture of components inserted in an extrusion chamber, forced by the action of at least one worm screw through the holes of a die placed on the head of the extruder and then cut by knives into the desired croquette form. The tablets are obtained starting from a mixture of components in powder form, via cold pressing, i.e. at a temperature close to ambient temperature.

The nutritional matrix comprising one or more protein hydrolysates is divided between the food croquettes and the therapeutic tablets, i.e. it is partially comprised in the croquettes and partially comprised in the tablets, and the phytotherapeutic extracts, including at least Piper nigrum, as well as the possibly present zinc, are completely contained in the therapeutic tablets.

In particular the protein hydrolysates are advantageously contained in the tablets of the product for feeding dogs according to the invention in a percentage by weight comprised between 1.5% and 4% and preferably comprised between 2% and 2.5%. The remaining quantity of protein hydrolysates provided in the product for feeding dogs according to the invention is contained in the food croquettes. The fact that the phytotherapeutic extract or the phytotherapeutic extracts, as well as the possible zinc, are contained in tablets obtained via cold pressing and not in croquettes obtained via extrusion ensures that the phytotherapeutic extract or the phytotherapeutic extracts themselves, as well as the possible zinc, are not subjected to thermal stresses during the process of production of the product for feeding dogs according to the invention, given that they are not subjected to temperatures greater than about 25-30°C during the entire production process; hence, the full maintenance of the functional properties of the phytotherapeutic extracts and/or of the trace element themselves is ensured, without the active principles supplied thereby being altered by the production process.

Preferably the product, subject of the present invention, has a moisture content of less than 15%, such that oxidative phenomena of the phytotherapeutic extracts contained therein are not facilitated during the period of preservation of the product itself.

Preferably, the therapeutic tablets are contained in the product according to the invention in a percentage by weight comprised between 5% and 15% and in particular of about 7%.

In particular, the product according to the present invention is advantageously made available on the market in packaged form, with the croquettes and the tablets that constitute it already mixed together inside suitable packages. The availability of a complete product, capable of supplying the dog with all the nutrients required as well as with the active principles having therapeutic properties, makes it particularly easy to administer the latter to the dog with his diet.

Advantageously the product for feeding dogs according to the present finding has an overall complete nutritional profile, formed by at least proteins, fats, carbohydrates, dietary fiber and ashes, wherein the proteins are preferably in a quantity by weight comprised between 15% and 40%, the fats are preferably in a quantity by weight comprised between 5% and 25%, the carbohydrates are preferably in a quantity by weight comprised between 18% and 65%, the dietary fiber is preferably in a quantity by weight comprised between 1.5% and 7% and the ashes are preferably in a quantity by weight less than 10%.

Such nutritional profile has proven particularly suitable for supplying all the nutrients required to dogs administrated the product, in a balanced manner, facilitating the increase of lean mass in such dogs, to the detriment of fat mass, with the previously specified consequent advantages.

For the above-listed grounds, the product for feeding dogs, subject of the present invention, has proven suitable for performing an optimal immunomodulation action and is adapted for being advantageously employed for feeding dogs affected by leishmaniasis as well as for feeding dogs more generally affected by pathologies that involve the immune system or by neoplasms. In particular, the product for feeding dogs according to the invention is indicated in the diet of dogs affected by Lyme disease, by Piroplasmosis, by Ehrlichiosis or by any disease that involves the immune system. In addition, the product in accordance with the present invention is particularly indicated in the diet of dogs affected by intestinal parasitosis or of dogs that have contracted external parasites, such as fleas, ticks or mites, in order to facilitate the eradication and/or control of such internal or external parasites.

The above-described formulation of the product, subject of the invention, has proven to be effective also towards other pathologies that involve the immune system, in particular with reference to dogs but also regarding cats. In particular, clinical studies have shown the remission of the symptoms associated with keratoconjunctivitis sicca (KCS) following the administration of a diet based on the present product.

In general, therefore, the product for feeding dogs according to the present invention is adapted for being administrated to subjects who require strengthening their immune defenses.

Hence, the product according to the invention is particularly indicated for the diet of dogs whose immune system is particularly exhausted or weakened, for example during nutritional recovery and convalescence or for reducing food intolerances, or for the prevention and treatment of the oxidative stress that can be manifested as stress from heat in some predisposed breeds.

Generally, therefore, the product for feeding dogs, subject of the present invention, is adapted for being advantageously employed in order to subject dogs affected by infectious, autoimmune and/or parasitic diseases to a curative diet, where the product according to the invention acts as an adjuvant of specific pharmacological therapies - in order to improve, in the dogs administrated with such product, the immune response to the pathology or to the pathologies affecting such dogs. As demonstrated above, the product for feeding dogs, subject of the present finding, has proven particularly useful as adjuvant in the treatment of leishmaniasis.

The same product is also adapted for being advantageously employed in a preventive diet, given that it has proven to be particularly effective in reinforcing the immune defenses of the dogs to which it is administrated, and thus proven adapted for decreasing the probability that the same contract and/or develop one or more of the abovementioned pathologies.

It is important to underline that the product for feeding dogs according to the present invention does not act as a generic immunostimulant, i.e. it does not enhance in an undifferentiated manner the immune response of the dogs that assume it, but it intervenes on such immune response, modulating it, i.e. in particular reducing the humoral or antibody response (Th2) in favor of the cell-mediated immune response (Th1).

### Clinical trials

By way of a non-limiting example, two clinical trials are described below that were conducted on dogs of different breeds, sex and age, affected by leishmaniasis and the relative obtained results.

In order to evaluate the effectiveness of the product for feeding dogs according to the present invention, as adjuvant in anti-leishmaniasis therapy, several biochemical parameters were measured in the subjects subjected to the clinical trials correlated with the immune response thereof, and the variation of the aforesaid parameters was evaluated over time, as is better explained hereinbelow.

The clinical trials described below were carried out by employing the following particular composition of the product for feeding dogs according to the present invention (in which the quantity of each component is reported in percentage by weight of the product):
- percentage by weight of protein hydrolysates (protein source) comprised between 7% and 14%;
- percentage by weight of Piper nigrum comprised between 0.0020% and 0.0030%;
- percentage by weight of herring flour (protein source) comprised between 4% and 14%;
- percentage by weight of fish oil (lipid source) comprised between 2% and 8%;
- percentage by weight of corn oil (lipid source) comprised between 0.5% and 2%;
- percentage by weight of rice flour (glucide source) comprised between 20% and 60%;
- percentage by weight of alga (fiber source) comprised between 4% and 7%;
- percentage by weight of beer yeast (mixed source) comprised between 0.5% and 4%;
- percentage by weight of mineral substances (mineral sources) comprised between 0.1% and 5%.

### First clinical trial:

A first clinical trial was aimed to verify the immunomodulation effectiveness of the product for feeding dogs according to the present invention and the effectiveness of the same product as adjuvant in the standard pharmacological therapy for the treatment of canine leishmaniasis, with respect to a standard diet.

In accordance with such first clinical trial, a first group of dogs (Group I), naturally infected by leishmania infantum, were subjected for six months to a pharmacological treatment with meglumine antimoniate and allopurinol and to an exclusive diet treatment with the product for feeding dogs subject of the present invention.

A second group of dogs (Group II), also naturally infected by leishmania infantum, were subjected for six months to a same pharmacological treatment with meglumine antimoniate and allopurinol and to a standard diet.

A third and a fourth group of clinically healthy dogs, i.e. with negative results of the serological, parasitological and molecular examinations, were used as control groups; the dogs of such groups were subjected to the diet respectively provided for the first and the second group of subjects.

In the subjects of the different groups, the blood concentration of CD4+ and CD8+ T lymphocytes and pro-inflammatory T cells were measured, by means of known antibody analyses usually employed for such purpose, at the time of the start of the trial, after three months of treatment and after six months of treatment.

The obtained results are summarized in the diagrams reported in the enclosed Figures 1-3a. More in detail, it is possible to observe in the enclosed graphs that at the start of the trial both Groups I and II of dogs had a considerable reduction of the Treg cells in the peripheral blood, as is visible in Figures 1 and 1a, and that there was a considerable increase of the percentage of Treg cells in Group I after 3 and 6 months of treatment, reaching percentages of Treg comparable to those measured in healthy dogs.

For the purpose of verifying if the immunological profile encountered in the treated dogs was linked to the introduction of a specific cytokine profile in the T lymphocytes, the production of IFN-γ and IL-4 in the T lymphocytes of both Groups I and II was analyzed. As is visible in Figures 2 and 2a, at the time of the start of the trial, the percentage of Th1 cells, which produce IFN-γ and are instead negatively correlated with IL-4, was slightly greater in the infected dogs than in the healthy dogs. As is visible in Figure 2, the percentage of Th1 cells showed a growth trend only in the dogs of Group I, leading to a significant increase of the Th1 cells in the dogs of the latter group after six months of treatment, while an analogous increase of Th1 cells was not verified in the dogs of Group II, not even at the end of the six months of treatment, as is visible in Figure 2a. It was also interesting to observe that the production of IL-4 decreased only in Group I after six months of treatment, as is visible in Figure 3, while it considerably increased in the dogs of Group II after six months of treatment, as is visible in Figure 3a.

The results obtained in this first clinical trial demonstrate the immunomodulation properties of the product for feeding dogs according to the present invention. Indeed, it was verified that a diet based on the product for feeding dogs according to the present invention is capable of interfering with the profile of the Tregulatory cells, along with the secretion of pro-inflammatory cytokines. In particular the diet based on the product according to the invention has proven capable of enhancing the effects of the pharmacological treatment.

After three and six months of treatment, i.e. of administration of a pharmacological therapy accompanied by a diet based on the product for feeding dogs according to the invention, the subjects of Group I showed a significant recovery in the percentage of Treg cells, reaching values of such percentages comparable with those encountered in healthy dogs. The observation of such phenomenon, accompanied by the decrease of Th2 cells, can in particular be correlated with a decrease of the pro-inflammatory effects in the peripheral tissues.

### Second clinical trial:

A second clinical trial was carried out on dogs naturally affected by leishmaniasis, in symptomatic clinical form.

This second trial was in particular aimed to verify the effective synergistic value of the main components of the product for feeding animals according to the present invention adapted to perform an immunomodulation action on the organism of the dogs that assume the product itself, i.e. the protein hydrolysates and the phytotherapeutic extract of Piper nigrum.

For such purpose, in such second trial, a first group of dogs (Group I) was subjected to a controlled diet based on the product for feeding dogs in accordance with the present invention in its complete form, a second group of dogs (Group II) was subjected to a controlled diet based on a product for feeding dogs entirely similar to the product according to the present invention, but lacking the component of the protein hydrolysates, which were substituted with a conventional protein source, and a third group of dogs (Group III) was subjected to a controlled diet based on a product for feeding dogs entirely similar to the product according to the present invention, but lacking the component of the phytotherapeutic extract of Piper nigrum.

In combination with the relative diet, each of the abovementioned groups of dogs was subjected to the classic pharmacological therapy provided for the treatment of the leishmaniasis pathology and constituted by meglumine antimoniate and allopurinol at dosages normally employed in clinical practice.

For each of the dogs, the trial began on day 0 with the dogs not pharmacologically treated for at least 1 year and not subjected to a controlled and specific diet. The selected dogs were subjected on day 0 (T0) to a quantification of the interleukin 10 present in their serum in basal conditions and to a quantification of the serum leptin, always in basal conditions.

In order to quantify the interleukin-10 (IL-10) and the leptin in the serum of the collected samples, a direct (sandwich) E.L.I.S.A. immuno-enzymatic test was used. All the samples and standards were used in duplicate, with good repeatability. During the trial, after 3 months (T1) and after six months (T2) of treatment, the evaluations of the immunological profile of the dogs were once again executed by means of quantification of the interleukin 10 and of the serum leptin.

The results obtained for the three different groups of dogs were summarized in the graphs pursuant to Figure 4, with regard to the concentrations of leptin, and pursuant to Figure 5, with regard to the concentrations of interleukin 10. Reported by way of example in Table 1 are the values of the leptin and IL-10 concentrations specifically for four subjects of each of the desired groups.

The results obtained in such second experimental trial show that in the subjects which received a diet based on the product according to the present invention, including the protein hydrolysates and the phytotherapeutic extract of Piper nigrum, there was a progressive decrease of the serum leptin, in accordance with a similar progressive increase of the quantity of interleukin 10 present in the serum. The progression of these two parameters, that was however not verified in the subjects of Groups II and III that did not receive the product for feeding dogs in its complete form, as clearly visible in the enclosed Figures 4 and 5, demonstrates the immunomodulation capacities of the product according to the invention.

The clinical evaluation of the subjects of Group I also allowed indicating in the latter a clear improvement of the lean mass/fat mass ratio, in favor of the first, accompanied by a good improvement of the general clinical conditions - not encountered in equal measure in the subjects of Groups II and III.

Indeed, in the trial subjects that instead received the diet lacking one of the two abovementioned components (Groups II and III), the clinical conditions did not show signs of clear improvement, as confirmed by laboratory data relative to the leptin and IL-10 serum concentrations, which presented statistically insignificant oscillations neither towards an increase nor towards a decrease thereof.

The finding thus conceived therefore attains the pre-established objects.

## Claims

1. Product for feeding dogs suitable for performing an immunomodulation action as adjuvant in a standard pharmacological therapy for the treatment of canine leishmaniasis, which product comprises a nutritional matrix and at least one phytotherapeutic extract, **characterized in that** said nutritional matrix comprises at least one protein hydrolysate, contained in said product for feeding dogs in a percentage by weight comprised between 7% and 14%, and said at least one phytotherapeutic extract comprises a phytotherapeutic extract of Piper nigrum comprised in said product for feeding dogs in a percentage by weight comprised between 0.0015% and 0.0035%;
wherein said at least one protein hydrolysate comprises fish protein hydrolysate.

2. Product for feeding dogs according to claim 1, **characterized in that** said phytotherapeutic extract of Piper nigrum is comprised in said product for feeding dogs in a percentage by weight equal to about 0.0025%.

3. Product for feeding dogs according to any of the preceding claims, **characterized in that** said phytotherapeutic extract of Piper nigrum is a dry extract titrated at 95% piperine.

4. Product for feeding dogs according to any of the preceding claims, **characterized in that** said at least one phytotherapeutic extract comprises, in addition to said phytotherapeutic extract of Piper nigrum, one or more further phytotherapeutic extracts selected from among phytotherapeutic extracts of: melon, soy, echinacea, aloe, fermented papaya, pomegranate and green tea.

5. Product for feeding dogs according to any of the preceding claims, **characterized in that** it comprises zinc.

6. Product for feeding dogs according to any of the preceding claims, **characterized in that** it comprises food croquettes, in particular obtained via extrusion, and therapeutic tablets obtained via cold compression from powders, said nutritional matrix comprising at least one protein hydrolysate being divided between said food croquettes and said therapeutic tablets and said at least one phytotherapeutic extract being contained in said therapeutic tablets.

7. Product for feeding dogs according to claim 6, **characterized in that** said therapeutic tablets are contained in said product in a percentage by weight comprised between 5% and 15% and preferably about 7%.

8. Product for feeding dogs according to claim 6 or 7, **characterized in that** it has an overall nutritional profile formed by at least proteins, fats, carbohydrates, dietary fiber and ashes, wherein the proteins are in a quantity by weight comprised between 15% and 40%, the fats are in a quantity by weight comprised between 5% and 25%, the carbohydrates are in a quantity by weight comprised between 18% and 65%, the dietary fiber is in a quantity by weight comprised between 1.5% and 7% and the ashes are in a quantity by weight less than 10%.

## Patentansprüche

1. Hundefutter zur Herbeiführung einer Immunomodulationswirkung als Hilfsstoff in einer pharmakologischen Standardtherapie zur Behandlung von Leishmaniose bei Hunden, wobei das Produkt eine Nährstoffmatrix und mindestens einen phytotherapeutische Extrakt umfasst, **dadurch gekennzeichnet, dass** die Nährstoffmatrix mindestens ein im benannten Hundefutter enthaltenes Proteinhydrolysat in einem Gewichtsprozentsatz zwischen 7 % und 14 % umfasst und der benannte mindestens eine phytotherapeutische Extrakt einen im benannten Hundefutter enthaltenen phytotherapeutischen Extrakt von Piper nigrum in einem Gewichtsprozentsatz zwischen 0,0015 % und 0,0035 % umfasst;
worin das benannte mindestens eine Proteinhydrolysat Fischproteinhydrolysat umfasst.

2. Hundefutter nach Anspruch 1, **dadurch gekennzeichnet, dass** der benannte phytotherapeutische Extrakt von Piper nigrum im benannten Hundefutter in einem Gewichtsprozentsatz von ca. 0,0025 % enthalten ist.

3. Hundefutter nach irgendeinem der vorangegangenen Ansprüche, **dadurch gekennzeichnet, dass** es sich bei dem benannten phytotherapeutischen Extrakt von Piper nigrum um einen bis 95 % Piperin titrierten Trockenextrakt handelt.

4. Hundefutter nach irgendeinem der vorangegangenen Ansprüche, **dadurch gekennzeichnet, dass** der benannte mindestens eine phytotherapeutische Extrakt zusätzlich zu dem benannten phytotherapeutischen Extrakt von Piper nigrum einen oder mehrere unter phytotherapeutischen Extrakten von Melone, Soja, Echinacea, Aloe, fermentierter Papaya, Granatapfel und Tee ausgewählte weitere phytotherapeutische Extrakte umfasst.

5. Hundefutter nach irgendeinem der vorangegangenen Ansprüche, **dadurch gekennzeichnet, dass** es Zink umfasst.

6. Hundefutter nach irgendeinem der vorangegangenen Ansprüche, **dadurch gekennzeichnet, dass** es insbesondere durch Extrusion hergestellte Futterkroketten und durch Kaltpressung aus Pulver hergestellte therapeutische Tabletten umfasst, wobei die benannte Nährstoffmatrix mindestens ein zwischen den benannten Futterkroketten und den benannten therapeutischen Tabletten aufgeteiltes Proteinhydrolysat und mindestens einen in den benannten therapeutischen Tabletten enthaltenen phytotherapeutischen Extrakt umfasst.

7. Hundefutter nach Anspruch 6, **dadurch gekennzeichnet, dass** die benannten therapeutischen Tabletten in dem benannten Futter in einem Gewichtsprozentsatz zwischen 5 % und 15 % und vorzugsweise ca. 7 % enthalten sind.

8. Hundefutter nach Anspruch 6 oder 7, **dadurch gekennzeichnet, dass** es ein mindestens aus Proteinen, Fetten, Kohlenhydraten, Ballaststoffen und Asche bestehendes Gesamtnährstoffprofil aufweist, worin die Proteine in einer Gewichtsmenge zwischen 15 % und 40 %, die Fette in einer Gewichtsmenge zwischen 5 % und 25 %, die Kohlenhydrate in einer Gewichtsmenge zwischen 18 % und 65 %, die Ballaststoffe in einer Gewichtsmenge zwischen 1,5 % und 7 % und die Asche in einer Gewichtsmenge von weniger als 10 % vorliegen.

## Revendications

1. Nourriture pour chiens permettant de réaliser une action d'immunomodulation comme adjuvant dans une thérapie pharmacologique pour le traitement de la leishmaniose canine, cette nourriture comprenant une matrice alimentaire et au moins un extrait phytothérapique, **caractérisée en ce que** ladite matrice alimentaire comprend au moins un hydrolysat de protéine, contenu dans ladite nourriture pour chiens en pourcentage par poids compris entre 7 % et 14 %, et ledit au moins un extrait phytothérapique comprend un extrait phytothérapique de Piper nigrum compris dans ladite nourriture pour chiens en pourcentage par poids compris entre 0,0015 % et 0,0035 % ;
où au moins un hydrolysat de protéine comprend un hydrolysat de protéine de poisson.

2. Nourriture pour chiens selon la revendication 1, **caractérisée en ce que** ledit extrait phytothérapique de Piper nigrum est compris dans ladite nourriture pour chiens en pourcentage par poids équivalent à environ 0,0025 %.

3. Nourriture pour chiens selon l'une quelconque des revendications précédentes, **caractérisée en ce que** ledit extrait phytothérapique de Piper nigrum est un extrait sec titré en piperine à 95 %.

4. Nourriture pour chiens selon l'une quelconque des revendications précédentes, **caractérisée en ce que** ledit au moins un extrait phytothérapique comprend en plus dudit extrait phytothérapique de Piper nigrum, un ou plusieurs extraits phytothérapiques sélectionnés parmi des extraits phytothérapiques de : melon, soja, échinacée, aloès, papaye fermentée, grenade et thé vert.

5. Nourriture pour chiens selon l'une quelconque des revendications précédentes, **caractérisée en ce qu'**elle comprend du zinc.

6. Nourriture pour chiens selon l'une quelconque des revendications précédentes, **caractérisée en ce qu'**elle comprend des croquettes alimentaires, en particulier obtenues par extrusion, et des comprimés thérapeutiques obtenus par compression à froid à partir de poudres, ladite matrice alimentaire comprenant au moins un hydrolysat de protéine étant divisé entre lesdites croquettes alimentaires et lesdits comprimés thérapeutiques et ledit au moins un extrait phytothérapique étant contenu dans lesdits comprimés thérapeutiques.

7. Nourriture pour chiens selon la revendication 6, **caractérisée en ce que** lesdits comprimés thérapeutiques sont contenus dans ladite nourriture en pourcentage par poids compris entre 5 % et 15 %, préférablement 7 % environ.

8. Nourriture pour chiens selon la revendication 6 ou 7, **caractérisée en ce qu'**elle a un profil alimentaire global formé par au moins des protéines, des lipides, des glucides, des fibres alimentaire et des cendres, oùles protéines sont en quantité par poids comprise entre 15 % et 40 %, les lipides sont en quantité par poids comprise entre 5 % et 25 %, les glucides sont en quantité par poids comprise entre 18 % et 65 %, les fibres alimentaires sont en quantité par poids comprises entre 1,5 % et 7 % et les cendres sont en quantité par poids inférieure à 10 %.
